# EUROPEAN PATENT APPLICATION

(11) **EP 3 617 705 A1**
(43) Date of publication of application: **04.03.2020**
(21) Application number: 18192215.4
(22) Date of filing: 03.09.2018
(51) Int. Cl.: G01N 33/02, G01N 33/04, G01N 31/22, G01N 33/52, G01K 3/04

(54) **POST-OPEN FRESHNESS INDICATING PACKAGE**

(71) Applicant: Tetra Laval Holdings & Finance S.A., 1009 Pully (CH)
(72) Inventor: STÅHLBERG, Jennie, 21851 Klagshamn (SE); SICKERT, Lars, 211 15 MALMÖ (SE); LEVANDER, Gustav, 24533 Staffanstorp (SE); HURDALEK, Ladislav, 22581 Lund (SE)
(74) Representative: Tetra Pak - Patent Attorneys SE

(57) **Abstract**

The present invention relates to a food package (1), comprising a closure (2), a package body (3), and a freshness indicator (4). The present invention further relates to a method (10) of evaluating the quality of a food in the food package, and a system (20) for real-time monitoring spoilage degree of package food.

## Description

### Field of the invention

The present invention provides a food package able to indicate post-opening decrease in food freshness. It is provided a way to detect spoilage of a food product.

### Background

The quality and safety of foods are key priorities for the food industry and the authorities. Thus, measures to assure this are increasing. Packages for protecting and transporting food during are commonly used. On many packages a "best before" date or expiration date are provided. It is well known that huge amounts of foods are discarded by consumers purely based on said dates, which foods many times could actually be consumed without problems.

On food packages an advice about "consume by" from opening may be provided. The current advice on food packages may be to consume a product within e.g. two days of opening and to keep refrigerated. This may for some products seem like a short period of time, however it has been found that many household refrigerators run at too high temperatures. If products actually are kept in good conditions the expiry date have a buffer time period, and can hence be used several days extra - if kept properly. Therefore, the "best before" date, or expiration date, or "post-opening consume by" advice provided needs to be overly cautious.

A further complication for the consumer is absent-mindedness. By forgetting when you opened the package and forgetting to use the product while it is still fresh, the food may too early become household food waste.

At present, any expiration date or a "best before" date is set according to the date of production. However, when the package is opened and subsequently stored may have a significant influence on the food's spoilage rate.

Many types of foods have been around for centuries. Thus, a consumer very often knows and recognizes the smell, look or taste of e.g. spoiled milk, butter, meat etc. However, the food industry continuously develops and market new products which are unfamiliar to consumers nowadays, of which consumers have no relation to in view of smell, look, or taste. Thus, a consumer may have serious problems to with its sense of smell or taste, or by visual inspection estimate the freshness of such products as the consumer has no frame of reference to such products. Many people are sensitive to dairy products and others may be requesting more plant based foods for improved sustainability of the world's resources. Thus, the industry e.g. provides soy or oat-based milk-like, butter-like, yoghurt-like, and cream-like products. However, it is very difficult for a consumer to know what such products would look, taste, or smell like upon spoilage. Therefore, it has been found that consumers may discard such products way before any "best before" or expiration date due to unawareness and a general fear of consuming spoiled products. On the contrary, the products may go bad quicker post-opening depending on handling, and in such cases the consumer may unknowingly consume foods gone bad, which the risk of possible health complications.

It is desirable to provide a more sustainable approach to food freshness and food waste, which makes it easier for a consumer to avoid premature rejection of foods, and to avoid possible health related effects due to consumption of spoiled foods.

### Summary of the invention

The present invention provides a package suited for increasing consumer awareness of product freshness.

It is herein provided a package that reports in an individual, nondestructive way and in real-time the quality and safety of food post-opening.

The present invention provides a food container provided with a post-opening food freshness indicator. The indicator may be provided to the food container's outer surfaces or may be integrated into the structures of the food container. The present indicator provides a visual statement of the contained food's current state, i.e. the freshness, post opening, for a viewer or consumer. The present invention may provide a way which could actually extend the days of use of food products, e.g. by also include the buffer time period of the expiry date, or beyond, depending on handling post-opening. If the food product would be subjected to prolonged undesirable conditions, the present invention would assist in providing an indication of decreased consumption period in relation to the expiry date provided on the food package by the producer. A more accurate indication of freshness is provided and will often save a consumer throwing out food unnecessarily while it is still safe to eat.

The present invention provides a possibility for the consumers to make more informed decisions about food freshness.

In one aspect the present invention relates to a food package comprising a closure, a package body, and a freshness indicator. The freshness indicator may be positioned partially in or on the closure and partially on the package body. The freshness indicator may comprise an indication section, wherein said indication section is provided in or on the closure, or on the package body. The freshness indicator may be adapted to be activated by opening the closure of the package. The freshness indicator may be adapted to be activated by:
i) applying a pressure to said freshness indicator,
ii) pulling a tab from said indicator,
iii) tearing or cutting a layer, preferably polymeric layer or fabric, of the indicator,
upon opening the closure. The closure may be adapted to be opened by a motion; preferably a popping motion, a pulling motion, a screwing motion, a twisting motion, a push-and-twist motion, a squeeze-and-pull motion, a squeeze-and-push motion, a pull-and-squeeze motion, a push-and-squeeze motion. The indication section of the freshness indicator may comprise an indicator composition, which may be selected from a liquid or a solid. The indicator composition may provide a physical or chemical change. The indicator composition may be:
a) adapted for a change of colour;
b) adapted for provision of an image in the indication section; or
c) adapted for provision of migration through a porous substrate or polymeric matrix or membrane, to allow a viewer to see a colour change or image formation of the freshness indicator; or
d) adapted for provision of a tactile change in the indication section. The indicator composition may be adapted to migrate at least partially through the porous substrate or polymeric matrix or membrane to show the change in colour or image formation of the freshness indicator. The indicator composition may be adapted to change appearance when exposed to at least one of: the atmosphere in or around the package, at least a predetermined temperature, predetermined time, and any combination thereof. The food freshness indicator may be adapted for detection of at least one of: pressure increase, gaseous substances comprising sulphur-containing compounds, carbon dioxide, and any combination thereof. The food freshness indicator may be adapted to change colour slower when stored at a lower temperature. The freshness indicator may comprise a time-temperature indicator. The time-temperature indicator may be selected from a chemical dissolution, a diffusion, an enzymatic, or a polymerization-based indicator. The indicator composition is contained in a receptacle, which is ruptured upon activation. The closure may be selected from the group consisting of cap, lid, pour spout, weld, seam; preferably screw cap. The closure may be mounted in an upper part of said package body; preferably atop or adjacent to said top of the package. The closure, preferably cap, may be transparent or comprises a transparent section to allow visibility of the freshness indicator positioned on the inside of or incorporated into said closure. The package may preferably comprise a liquid product or semi-liquid product. The food package may be adapted for dairy products, such as milk, yoghurt, and cream; juices; fruit preserves; dairy substitution products such as almond milk, soy and/or oat containing products such as oat/soy drinks like oat/soy milk, oat/soy yoghurts, oat/soy cream; sauces; or broths.

In one aspect the present invention relates to a method of evaluating the quality of a food in a food package comprising:
packaging a food product in a package,
providing a freshness indicator to the package, unless already present in or on the package, to provide the food package according to the present invention containing food,
activating said freshness indicator by opening the food package,
inspecting the freshness indicator at least once to determine if the food package has been subjected to an undesirable exposure, preferably time or time-temperature, above a predetermined value/threshold that increase the likelihood of spoilage of said food product.

In one aspect the present invention relates to a system for real-time monitoring spoilage degree of packaged food comprising:
a food package according to the present invention,
a camera or vision system adapted to record the colour or image change of the indicator on said food package, and
a control unit. The control unit may have a database, a file, a data processor for receiving data and correlate the received data on visual colour or image change with at least a predetermined set point colour or image value and displaying for a user connected to the network. The control unit may be connected to a remote control unit via a network, the remote control unit having user interface capability, so that real time data of the deterioration process may be transferred from the control unit to be displayed for a user being connected to the remote control unit via the network. The control unit may further correlate the received data with an auto replenishment system to provide shopping list input, or a recepie search system for recipie suggestions. The remote control unit may be located in a cloud-based computing environment. The remote control unit may be a virtual control unit. The remote control unit may have a database, a file, a data processor for receiving data and correlate the received data on visual colour or image change with at least a predetermined set point colour or image value and displaying for a user connected to the network. The control unit may have a user interface capability, so that real time data of the deterioration process may be displayed for a user being connected to the control unit via the network. The remote control unit may further correlate the received data with an auto replenishment system to provide shopping list input, or a recepie search system for recipie suggestions.

### Short description of the drawings

Figure 1 discloses an embodiment of a food package according to the present invention with a freshness indicator.
Figure 2 discloses a schematic chart of a method of evaluating the quality of a food in a food package according to the present invention with a freshness indicator.
Figure 3 discloses a schematic block diagram of a system for real-time monitoring spoilage degree of packaged food according to the present invention.

### Detailed description

The present invention provides a visual or tactile indication of the expiry status of the product within a package from the time of opening and onwards.

When the food within a sealed container starts to spoil, irrespectively of it having been previously opened or not, several by-products may be given off. It may therefore be possible to detect spoilage by detecting one or more of such by-products. Common to all such deterioration is the production of pressure, gaseous sulphur-containing compounds, and carbon dioxide.

"Freshness" as used herein is to be interpreted as the consumer experience of the product with an actual expiry date connection to the food packaged. The specific contained food is often limiting when defining the freshness of a food. For example, the freshness of a food may be defined by colour changes, taste changes, fermentation action, decreased nutrition value, or potentially harmful upon digestion. Package foods today are labeled with expiry dates based on by the producer expected expiry date under specific temperature conditions and including a buffer time period. The producer may include considerations regarding product and consumption expectations for a consumer when they decide upon an expiry date. For juices, the vitamin content is critical, thus the degradation of vitamins trigger an expiry date labeling. As can be understood there is different perceptions of freshness depending on food product. However, that is not the sole truth regarding freshness, also cultural differences impact on a consumer's expectations. Different parts of the world rate foods differently. For example, a brown stained banana would in some countries be considered undesirable to buy and eat would in other areas of the world be considered soon to be ripe. As for fermented products such as yoghurts, that may be considered fresh but if a cream or milk would have the same fermented properties it would be considered past expiration date. Thus, there is no uniform definition of food freshness for all foods in all parts of the world. Freshness of a food is to be considered based on the perception and expectation by a consumer of a specific food product in question. Thus, the present food freshness indicator is to illustrate the perception and expectation by a consumer of a specific food product in question.

"Package body" as used herein is to be interpreted as the part of the package enclosing the food product. It is the part of the food package not being the closure.

In one aspect the present invention provides a food package, comprising a closure, a package body, and a freshness indicator. The freshness indicator is adapted to provide a post-opening colour change, image change or tactile change over time. The post-opening colour change, image change or tactile change may be affected and provided in different manners.

The freshness indicator may be positioned partially in or on the closure and partially on the package body.

The freshness indicator may comprise an indication section, wherein said indication section is provided in or on the closure, or on the package body. The indication section of the freshness indicator is adapted to visually indicate the freshness of the food contained within the package to a viewer.

The freshness indicator may be adapted to be activated by opening the closure of the package. The freshness indicator may be adapted to be activated by:
i) applying a pressure to said freshness indicator,
ii) pulling a tab from said indicator,
iii) tearing or cutting a layer, preferably polymeric layer or fabric, of the indicator,
upon opening the closure.

In this way the atmosphere may interact with the construction of the freshness indicator, or the freshness indicator may this way trigger a change of the appearance of a composition or allow migration of a composition within said indicator.

The closure may be adapted to be opened by a motion. Such a motion may be selected from the group consisting of a popping motion, a pulling motion, a screwing motion, a twisting motion, a push-and-twist motion, a squeeze-and-pull motion, a squeeze-and-push motion, a pull-and-squeeze motion, and a push-and-squeeze motion. The relative motion during opening causes activation by e.g. pressure, rupture, or pulling.

The indication section of the freshness indicator may comprise an indicator composition, which may be selected from a liquid or a solid.

The indicator composition may provide a physical or chemical change within the freshness indicator.

The indication section of the freshness indicator may change visual appearance or tactile appearance. A viewer or inspector may see a change in colour, a visualization of an image, or a change in tactile sensation. The change in appearance or feel may depend on the indicator composition.

The indicator composition may change visual appearance or tactile appearance. The indicator composition may change colour or provide an image in the indication section, e.g. by itself or upon interaction with other compounds, which may be due to different reactions; or the indicator composition, e.g. comprising an ink, is adapted for migration through a porous substrate or polymeric matrix or membrane, to allow a viewer to see a colour change or image formation of the indication section of the freshness indicator. Alternatively, the indication section may change tactile appearance. The tactile change of the indication section may be due to a transformation of the indicator composition, or a reaction connected to said indicator composition. The tactile change may be from a smooth surface to a surface with more tactile variation, such as protrusions, indentations, and may provide a bumpy surface or a rough surface such as a sand paper.

In this way a coloured indicator composition may be used which just slowly proceeds though a material until it becomes visible to a viewer of the freshness indicator. Alternatively, a coloured or clear indicator composition may be used which upon subjection to further components released by the activation of the freshness indicator may be visible to a viewer of the freshness indicator, or also slowly proceed through a material until it becomes visible to a viewer of the freshness indicator.

The indicator composition may be adapted to migrate at least partially through the porous substrate or polymeric matrix or membrane to show the change in colour, image or tactile feel of the freshness indicator, to denote the exposure of the food contained it is supported to reflect freshness of. The indicator composition may be released to or absorbed by the porous substrate or polymeric matrix or membrane. Thus, the indicator composition may slowly diffuse along the substrate, membrane or matrix to cause such a colour change, image change or tactile change.

The indicator composition may be adapted to change appearance when exposed to at least one of the atmosphere in or around the package, at least a predetermined temperature, predetermined time, and any combination thereof.

The food freshness indicator may be adapted to detect changes in atmosphere and/or temperature and correlate that to a time passed since activation. Opening the package breaks the freshness indicator and may change the atmosphere in the indicator, which may trigger a colour, image or tactile changing process. Marks or patterns of the freshness indicator will change colour, image appearance or tactile feel gradually from one status or colour to another over a couple of days, after which it is considered "past best". The change in appearance may be based on degradation of the components of the indicator composition. The indicator composition may comprise an ink. The colour or image change may be provided by a change in pH of the indicator composition providing acidic or alkaline degradation products which may be made visible by a pH indicator of the freshness indicator, preferably within the indicator composition.

Preferably the indicator composition is affecting a colour, image or tactile change of said indication section of the freshness indicator with time and temperature as such parameters are important the freshness of food. The indicator composition may comprise compounds selected from the group dyes; pH indicators, such as phenolphthalein, methyl red, bromothymol blue, and thymol blue; and oxygen responsive colour changing compositions, such as metal salts.

The food freshness indicator may be adapted for detection of at least one of pressure increase, gaseous substances comprising sulphur-containing compounds, carbon dioxide, and any combination thereof. Any one of these features may be an effect provided by microbial food spoilage.

The food freshness indicator may be adapted to change colour, image appearance or tactile feel slower when stored at a lower temperature, i.e. in a colder fridge. This would give a more accurate indication of freshness and will often save a consumer throwing out food unnecessarily while it is still safe to eat. The indicator is temperature sensitive, and optionally surrounding atmosphere sensitive, and will change colour, image or tactile apparence more slowly if the product is stored after opening at colder temperatures. Many household refrigerators run warmer than the recommended 4-5°C - this spoils fresh food faster. However, the indicator changes colour, image or tactile feel quicker to reflect a warmer storage temperature. The key is to keep your fridge at the correct temperature or below.

The freshness indicator may comprise a time-temperature indicator. By using a time-temperature indicator the cumulative time-temperature exposure may be reflected. However, freshness indicator may comprise a simple temperature indicator or time indicator in order to more simply reflect temperature history or time history alone. The freshness indicator may indicate the expiry status by colour, image or tactile change when subjected to undesirable temperature conditions, undesirable time durations, or any combination thereof.

If a time-temperature indicator is used it may be selected from a chemical dissolution, a diffusion, an enzymatic, or a polymerization-based indicator. Chemical dissolution may be including chemical dissolving of metals, such as aluminum.

The indicator composition of the freshness indicator may be contained in a receptacle, which is ruptured upon activation, e.g. opening of the closure of the package. Upon activation the indicator composition may be released or triggered for further reactions.

The closure of the package may be selected from the group consisting of cap, lid, pour spout, weld, and seam. The cap may be a screw cap. With a screw cap the freshness indicator is activated by the twisting motion performed to open the cap.

The closure is mounted in an upper part of said package body. Preferably the closure is positioned atop or adjacent to said top of the package.

The closure, preferably a cap, may be transparent, i.e. made of a transparent material, or comprises a transparent section to allow visibility of the freshness indicator positioned on the inside of or incorporated into said closure. It is desirable to show the change of appearance, i.e. colour or image change, to a viewer. Thus, it is desirable that at least parts of the closure such as cap is sufficiently transparent so that a change in appearance of the freshness indicator that results from exposure air, a temperature condition, or a time passed is detectable through the closure.

The freshness indicator may be applied to the cap and package body before or after filling the package with food product.

The indication section of the freshness indicator is preferably positioned so that it is visible to a viewer. When the closure is opened, the freshness indicator is preferably not torn or split at the indication section. However, the opening of the closure is done at a portion of the freshness indicator which at least influences the indication section, in order to start the freshness measurement.

The freshness indicator may be applied to the cap and package body by printing, sticker application, or mould labeling. If the indicator is mould labeled, the mould surface may need to be at least partially cooled not of negatively affect the freshness indicator, if it happens to be relying on temperature input. The freshness indicator may be applied in this manner before, during, or after filling of the package with food stuff. As an alternative the freshness indicator may be built into the closure of the package, e.g. during manufacturing of caps, wherein during the moulding of the caps a freshness indicator is incorporated therein. In such a case the freshness indicator would be positioned within the cap so that the indication section is visible to a viewer and another portion of the freshness indicator goes passed where the cap is opened e.g. by a popping or twisting motion, which motion would trigger the activation of the freshness indicator, to allow a change of colour, image or tactile appearance of the indication section for a viewer to assess.

In one embodiment the freshness indicator may be activated solely by pulling a tab or removing an outer layer, without a connection to opening the closure of the package. It is to be noted that the freshness indicator may be positioned on the closure of package body, on the package body, or on both the closure and the package body.

The present package may comprise a liquid or semi-liquid product. The food package may be adapted to contain dairy products, such as milk, yoghurt, and cream; juices; fruit preserves; plant based or dairy substitution products such as almond milk, soy and/or oat containing products such as oat/soy drinks like oat/soy milk, oat/soy yoghurts, oat/soy cream; sauces; or broths.

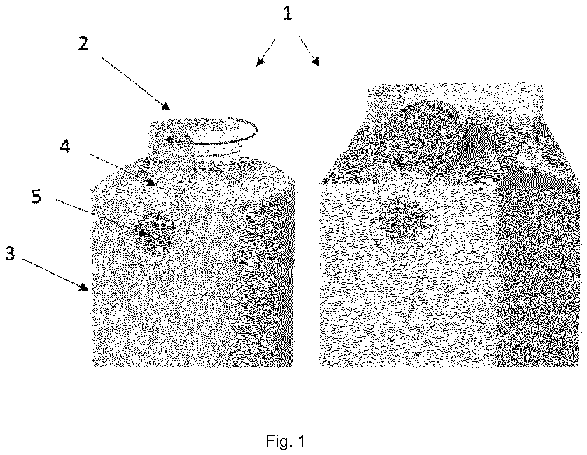

Figure 1 discloses a food package 1 having a closure 2, a package body 3, and a freshness indicator 4 comprising an indication section 5.

One aspect of the present invention provides a method of evaluating the quality of a food in a food package. The method comprising: packaging a food product in a package; providing the freshness indicator to the package, unless already present in or on the package, to provide the present food package containing food; activating said freshness indicator by opening the package; inspecting the freshness indicator at least once to determine if the package has been subjected to an undesirable exposure above a predetermined value/threshold that increase the likelihood of spoilage of said food product. The undesirable exposure may be reflected by time, time-temperature, pressure, carbon dioxide or gaseous sulphur-containing compounds indications of the freshness indicator.

The present freshness indicator may be inspected by a consumer by visual inspection by the naked eye. A further option could be that the freshness indicator could be monitored by other surveillance equipment. The surveillance equipment such as a camera or a vision system may enable a digital functionality by connecting to services related to product freshness via digital devices at home, in the fridge, or smart phones. This would allow food waste reduction by providing input on electronic shopping lists to indicate purchase in due time, suggest recipes based on expiration dates, etc.

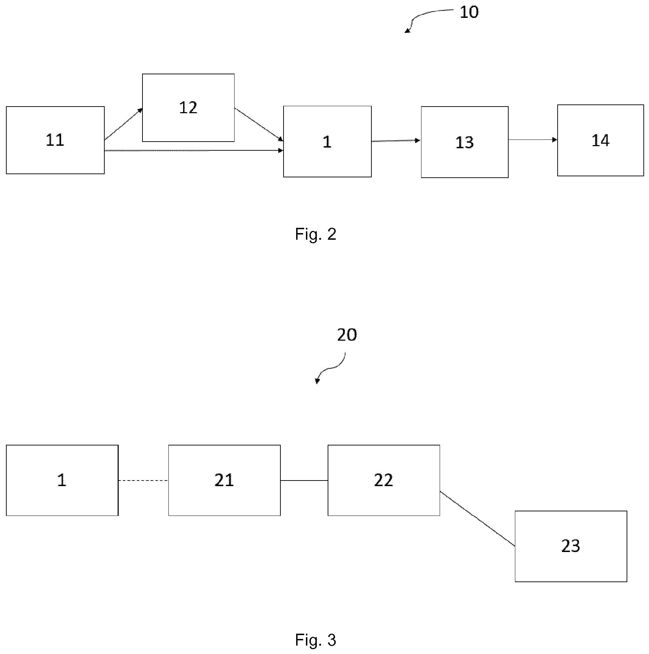

Figure 2 discloses an embodiment of the method of evaluating the quality of a food in a food package according to the present invention with a freshness indicator. The method comprises packaging 11 of a food product in a package, providing 12 a freshness indicator 4 to the package, unless already present in or on the package, to provide the food package 1 according the present invention containing food, activating 13 said freshness indicator 4 by opening the food package 1, inspecting 14 the freshness indicator 4 at least once to determine if the food package 1 has been subjected to an undesirable exposure, preferably time or time-temperature, above a predetermined value/threshold that increase the likelihood of spoilage of said food product.

One aspect of the present invention provides a system for real-time monitoring spoilage degree of packaged food comprising: the present food package, a camera or vision system adapted to record the colour or image change of the indicator on said package, and a control unit.

The control unit may have a database, a file, a data processor for receiving data and correlate the received data on visual colour or image change with at least a predetermined set point colour or image value and displaying for a user connected to the network.

The control unit may be connected to a remote control unit via a network, the remote control unit having user interface capability, so that real time data of the deterioration process may be transferred from the control unit to be displayed for a user being connected to the remote control unit via the network.

The control unit may further correlate the received data with an auto replenishment system to provide shopping list input, a recepie search system for recipie suggestions, or a loyalty program system for discount coupon retrieval.

The remote control unit may be located in a cloud-based computing environment. The remote control unit may be a virtual control unit.

The remote control unit have a database, a file, a data processor for receiving data and correlate the received data on visual colour or image change with at least a predetermined set point colour or image value and displaying for a user connected to the network.

The control unit having user interface capability, so that real time data of the deterioration process may be displayed for a user being connected to the control unit via the network.

The remote control unit may further correlate the received data with an auto replenishment system to provide shopping list input, a recepie search system for recipie suggestions, or a loyalty program system for discount coupon retrieval.

Figure 3 discloses an embodiment of the system for real-time monitoring spoilage degree of packaged food according to the present invention. The system 20 for real-time monitoring spoilage degree of packaged food comprises a food package 1 according to the present invention, a camera or vision system 21 adapted to record the colour or image change of the indicator 4 on said food package 1, and a control unit 22. The control unit 22 may be connected to a remote control unit 23 via a network.

From the description above follows that, although various embodiments of the invention have been described and shown, the invention is not restricted thereto, but may also be embodied in other ways within the scope of the subject-matter defined in the following claims.

## Claims

1. A food package (1), comprising a closure (2), a package body (3), and a freshness indicator (4).

2. The food package (1) according to claim 1, wherein said freshness indicator (4) is positioned partially in or on the closure (2) and partially on the package body (3).

3. The food package (1) according to claim 1 or 2, wherein said freshness indicator (4) comprises an indication section (5), wherein said indication section (5) is provided in or on the closure (2), or on the package body (3).

4. The food package (1) according to any one of claims 1-3, wherein said freshness indicator (4) is adapted to be activated by opening the closure (2) of the package (1).

5. The food package (1) according to claim 4, wherein the freshness indicator (4) is adapted to be activated by:
iv) applying a pressure to said freshness indicator (4),
v) pulling a tab from said indicator (4),
vi) tearing or cutting a layer, preferably polymeric layer or fabric, of the indicator (4),
upon opening the closure (2).

6. The food package (1) according to any one of claims 1-5, wherein said closure (2) is adapted to be opened by a motion; preferably a popping motion, a pulling motion, a screwing motion, a twisting motion, a push-and-twist motion, a squeeze-and-pull motion, a squeeze-and-push motion, a pull-and-squeeze motion, a push-and-squeeze motion.

7. The food package (1) according to any one of claims 1-6, wherein said indication section (5) of the freshness indicator (4) comprises an indicator composition, preferably selected from a liquid or a solid.

8. The food package (1) according to any one of claims 1-7, wherein said indicator composition provides a physical or chemical change.

9. The food package (1) according to any one of claims 1-8, wherein said indicator composition:
e) is adapted for a change of colour;
f) is adapted for provision of an image in the indication section; or
g) is adapted for provision of migration through a porous substrate or polymeric matrix or membrane, to allow a viewer to see a colour change or image formation of the freshness indicator; or
h) is adapted for provision of a tactile change in the indication section.

10. The food package (1) according to claims 9, wherein the indicator composition is adapted to migrate at least partially through the porous substrate or polymeric matrix or membrane to show the change in colour or image formation of the freshness indicator.

11. The food package (1) according to any one of claims 1-10, wherein said indicator composition is adapted to change appearance when exposed to at least one of:
the atmosphere in or around the package,
at least a predetermined temperature,
predetermined time, and
and any combination thereof.

12. The food package (1) according to any one of claims 1-11, wherein the food freshness indicator (4) is adapted for detection of at least one of:
pressure increase,
gaseous substances comprising sulphur-containing compounds, carbon dioxide, and
any combination thereof.

13. The food package (1) according to any one of claims 1-12, wherein the food freshness indicator (4) is adapted to change colour slower when stored at a lower temperature.

14. The food package (1) according to any one of claims 1-13, wherein the freshness indicator (4) comprises a time-temperature indicator.

15. The food package (1) according to claim 14, wherein the time-temperature indicator is selected from a chemical dissolution, a diffusion, an enzymatic, or a polymerization-based indicator.

16. The food package (1) according to any one of claims 1-15, wherein the indicator composition is contained in a receptacle, which is ruptured upon activation.

17. The food package (1) according to any one of claims 1-16, wherein said closure (2) is selected from the group consisting of cap, lid, pour spout, weld, seam; preferably screw cap.

18. The food package (1) according to any one of claims 1-17, wherein the closure (2) is mounted in an upper part of said package body; preferably atop or adjacent to said top of the package.

19. The food package (1) according to any one of claims 1-18, wherein the closure (2), preferably cap, is transparent or comprises a transparent section to allow visibility of the freshness indicator (4) positioned on the inside of or incorporated into said closure (2).

20. The food package (1) according to any one of claims 1-19, wherein the package (1) comprises a liquid product or semi-liquid product.

21. The food package (1) according to any one of claims 1-20, wherein adapted for dairy products, such as milk, yoghurt, and cream; juices; fruit preserves; dairy substitution products such as almond milk, soy and/or oat containing products such as oat/soy drinks like oat/soy milk, oat/soy yoghurts, oat/soy cream; sauces; or broths.

22. A method (10) of evaluating the quality of a food in a food package (1) comprising:
packaging (11) a food product in a package,
providing (12) a freshness indicator (4) to the package, unless already present in or on the package, to provide the food package (1) according to any one of claims 1-21 containing food,
activating (13) said freshness indicator (4) by opening the food package (1),
inspecting (14) the freshness indicator (4) at least once to determine if the food package (1) has been subjected to an undesirable exposure, preferably time or time-temperature, above a predetermined value/threshold that increase the likelihood of spoilage of said food product.

23. A system (20) for real-time monitoring spoilage degree of packaged food comprising:
a food package (1) according to any one of claims 1-21,
a camera or vision system (21) adapted to record the colour or image change of the indicator (4) on said food package (1), and
a control unit (22).

24. The system (20) according to claim 23, wherein the control unit (22) has a database, a file, a data processor for receiving data and correlate the received data on visual colour or image change with at least a predetermined set point colour or image value and displaying for a user connected to the network.

25. The system (20) according to claim 23 or 24, wherein the control unit (22) is connected to a remote control unit (23) via a network, the remote control unit (24) having user interface capability, so that real time data of the deterioration process may be transferred from the control unit (22) to be displayed for a user being connected to the remote control unit (23) via the network.

26. The system (20) according to any one of claims 23-25, wherein the control unit (22) may further correlate the received data with an auto replenishment system to provide shopping list input, or a recepie search system for recipie suggestions.

27. The system (20) according to claim 25 or 26, wherein the remote control unit (23) is located in a cloud-based computing environment.

28. The system (20) according to claim 27, wherein the remote control unit (23) is a virtual control unit.

29. The system (20) according to any one of claims 25-28, wherein the remote control unit (23) has a database, a file, a data processor for receiving data and correlate the received data on visual colour or image change with at least a predetermined set point colour or image value and displaying for a user connected to the network.

30. The system (20) according to any one of claims 23-29, wherein the control unit (22) having user interface capability, so that real time data of the deterioration process may be displayed for a user being connected to the control unit (22) via the network.

31. The system (20) according to any one of claims 25-30, wherein the remote control unit (23) may further correlate the received data with an auto replenishment system to provide shopping list input, or a recepie search system for recipie suggestions.
